Europäisches Patentamt
European Patent Office
Office européen des brevets

(11) Publication number: 0 209 814 A1

# EUROPEAN PATENT APPLICATION

(21) Application number: 86109550.3

(22) Date of filing: 11.07.86

(51) Int. Cl.⁴: C07D 215/56 , A61K 31/47

(30) Priority: 22.07.85 IT 2166285

(43) Date of publication of application: 28.01.87 Bulletin 87/05

(84) Designated Contracting States: AT BE CH DE FR GB IT LI LU NL SE

(71) Applicant: YASON S.r.l
Via Pisacane, 34/A
I-20129 Milan(IT)

(72) Inventor: Quadro, Giuseppe
Via Pisacane, 34/A
I-20129 Milan(IT)

(74) Representative: Bianchetti, Giuseppe
Studio Consulenza Brevettuale Via Rossini, 8
I-20122 Milan(IT)

## (54) Quinoline-3-carboxylic acid derivative, a process for the preparation thereof and pharmaceutical compositions containing it.

(57) 1-Ethyl-6-fluoro-1,4-dihydro-4-keto-7-(trimethylolmethylamino)quinoline-3-carboxylic acid of formula I

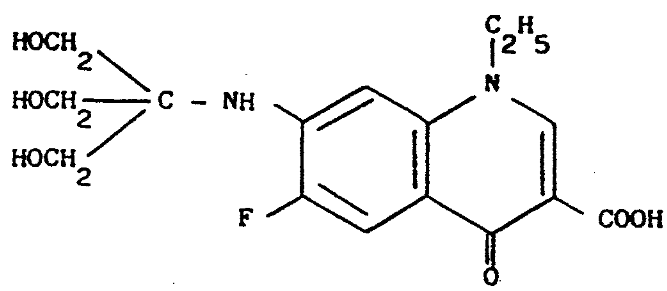

has such a valuable pharmacologic activity, to be used in human therapy.

EP 0 209 814 A1

**QUINOLINE-3-CARBOXYLIC ACID DERIVATIVE, A PROCESS FOR THE PREPARATION THEREOF AND PHARMACEUTICAL COMPOSITIONS CONTAINING IT**

The present invention relates to 1-ethyl-6-fluoro-1,4-dihydro-4-keto-7-(trimethylolmethylamino)quinoline-3-carboxylic acid, of formula I

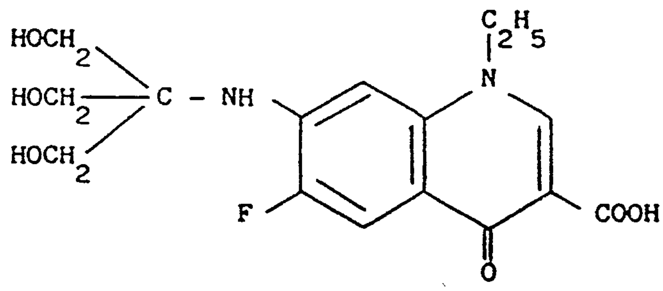

(I)

and to pharmaceutically acceptable salts thereof with non-toxic bases, such as sodium, ammonium, tromethamine, lysine, arginine, urotropin salts, etc.

The present invention relates also to a process for preparing compound I or salts thereof, and to pharmaceutical compositions having antibacterial activity, said compositions containing compound I or the salts thereof as the active ingredient.

Compounds being structurally similar to the compound according to the present invention are known: particularly norfloxacin, i.e. 1-ethyl-6-fluoro-1,4-dihydro-4-oxo-7-(1-piperazinyl)quinoline-3-carboxylic acid, described in Belgian Patent No. 870,917, is a well-known antibacterial agent, which is widely used in human therapy, mainly in the treatment of urinary infections.

However norfloxacin, though showing a remarkable antibacterial activity, suffers from pharmacokinetic restrictions (poor adsorption and bioavailability).

It has now been found that compound I, hereinafter defined YS 260 for sake of shortness, besides showing activity ant antibacterial spectrum substantially superimposable to those of norfloxacin, has remarkably better pharmacokinetic characteristics. Particularly, after administration of the above drugs in equimolecular dosages, higher hematic rates and accordingly a better bioavailability were evidenced for YS 260.

The better bioavailability of YS 260 allows to obtain the same therapeutic effectiveness of norfloxacin at reduced doses and accordingly an improved therapeutic index.

The plasmatic peak of YS 260 occurs about 90 minutes after the administration, in a 22.51 µg/ml maximum concentration, which is about 5 times higher than that obtained for similar quinolinone antibacterials.

Therefore YS 260 or the pharmaceutically acceptable salts thereof will be used as antibacterial drugs, particularly in the treatment of urinary infections (cystitis, pyelitis, cystopyelitis, pyelonephritis), by oral, parenteral or rectal administration, in form of such appropriate pharmaceutical compositions as capsules, tablets, sugar-coated pills, dragees, granulates, packets, syrups, solutions, injectable steril solutions, suppositories, etc.

Said dosage forms will contain therapeutically effective amounts of the active ingredient, in admixture with appropriate carriers, excipients, binding agents, preservatives, stabilizers, flavours, etc. traditionally used in pharmaceutical techniques.

The daily dosage will vary, depending upon pathology and conditions of the patient (weight, age, sex, etc.): it will generally range from 200 to 1000 mg, divided in 1-4 administrations. Therefore, unitary dosage forms will contain 100 to 500 mg of active ingredient.

The present invention also provides a process for preparing compound I, by reacting tris-hydroxymethylaminomethane and 1-ethyl-6-fluoro-1,4-dihydro-4-oxo-7-chloroquinoline-3-carboxylic acid - (which is a per se known compound) in aprotic solvents, such as dimethylsulfoxide, dimethylacetamide, dimethylformamide, in case in the presence of catalytic amounts of NaI. The reaction will be preferably carried out with an excess of tris-hydroxymethylaminomethane, at the reflux temperature of the solvent, for 12-24 hours.

The following Example further illustrates the invention, without limiting it.

EXAMPLE

300 Mg of 1-ethyl-6-fluoro-1,4-dihydro-4-keto-7-chloro-quinoline-3-carboxylic acid was dissolved in 15 ml of dimethylformamide. 3 g of tri-hydroxymethylaminomethane was added to the solution, which was refluxed for 16 hours, adding catalytic amounts of NaI. The reaction was checked by TLC, eluent: THF-ammonium hydroxide 25% (98-2), UV light; the spot of the chloroderivative disappears, while a new spot is evidenced, less eluted, fluorescent at 364 nm.

When the reaction was complete, the mixture was dried under vacuum, treated with acetic acid to adjust pH to 5, cooled to 0°C and filtered.

After crystallization (at least 2 times) from ethanol-water, cooling to 0°C before filtration, compound I was obtained, melting at 244°C, in a 25% yield.

## Claims

1. 1-Ethyl-6-fluoro-1,4-dihydro-4-keto-7-(trimethylolmethylamino)quinoline-3-carboxylic acid of formula I

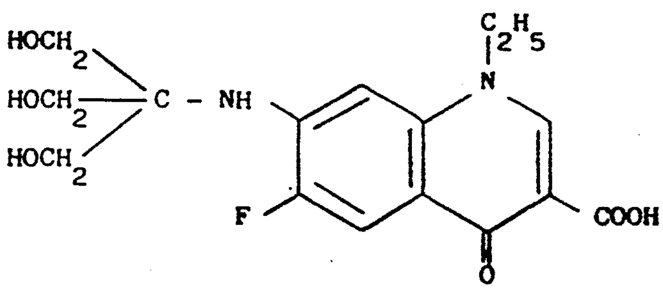

(I)

and pharmaceutically acceptable salts thereof with non-toxic organic and inorganic bases.

2. A process for preparing compound I, in which process 1-ethyl-6-fluoro-1,4-dihydro-4-oxo-7-chloro-quinoline-3-carboxylic acid is reacted with an excess of tris-hydroxymethylaminomethane.

3. A process as claimed in claim 2, which process is carried out in solvents such as dimethylsulfoxide, dimethylformamide, dimethylacetamide, at the reflux temperature of the solvent.

4. Pharmaceutical compositions having antibacterial activity, containing as the active ingredient compound I or one of its salts, in admixture with appropriate carriers or excipients.

5. Pharmaceutical compositions as claimed in claim 4, in form of capsules, tablets, sugar-coated pills, dragees, granulates, syrups, solutions, suppositories, vials.

Claims for contracting state AT

1. A process for preparing 1-ethyl-6-fluoro-1,4-dihydro-4-keto-7-(trimethylolmethylamino)quinoline-3-carboxylic acid of formula I

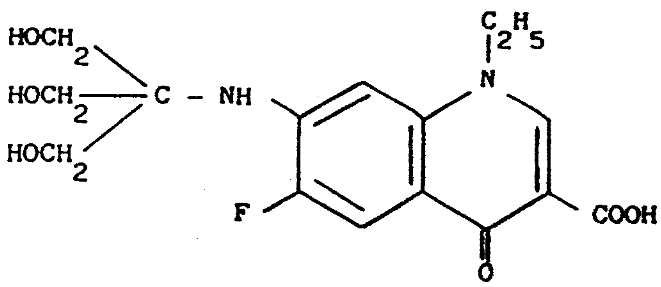

(I)

and pharmaceutically acceptable salts thereof with non-toxic organic and inorganic bases, in which process 1-ethyl-6-fluoro-1,4-dihydro-4-oxo-7-chloroquinoline-3-carboxylic acid is reacted with an excess of tris-hydroxymethylaminomethane.

2. A process as claimed in claim 1, which process is carried out in solvents such as dimethylsulfoxide, dimethylformamide, dimethylacetamide, at the reflux temperature of the solvent.

European Patent Office

# EUROPEAN SEARCH REPORT

Application number
EP 86109550.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A1 - 0 131 839 (ABBOT)<br>* Claims 1,9; example 38 (table III, No. 22) *<br>-- | 1,4 | C 07 D 215/56<br>A 61 K 31/47 |
| A | DE - A1 - 3 106 013 (KYORIN)<br>---- | | |
| | | | TECHNICAL FIELDS SEARCHED (Int. Cl.4) |
| | | | C 07 D 215/00 |

The present search report has been drawn up for all claims

| Place of search | Date of completion of the search | Examiner |
|---|---|---|
| VIENNA | 10-10-1986 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82